# EUROPEAN PATENT APPLICATION

(11) **EP 2 000 935 A2**
(43) Date of publication of application: **10.12.2008**
(21) Application number: 08153426.5
(22) Date of filing: 27.03.2008
(51) Int. Cl.: G06F 19/00

(54) **Method of processing protein peptide data and system**

(30) Priority: 10.05.2007 EP 07107948
(71) Applicant: F.HOFFMANN-LA ROCHE AG, 4070 Basel (CH)
(72) Inventor: Berntenis, Nikolaos, 68300 Saint Louis (FR); Miess, Christian, 79639 Grenzach-Wyhlen (DE); Mueller, Bernd, 82347 Bernried (DE)
(74) Representative: Vossius & Partner

(57) **Abstract**

The invention provides a method of processing protein peptide data obtained from healthy or pathological samples for analysis, comprising the steps of: providing a list of peptide sequences and associated auxiliary information representing an input data set; compiling from the input data set a new peptide sequence list by removing peptide sequence redundancy in the peptide sequence list, said new peptide sequence list representing a peptide data set; and grouping together members of the peptide data set originating from the same protein thus generating a protein data set.

## Description

The invention relates to protein identification and provides a method of and a system for processing protein peptide data, preferably obtained from healthy or pathological samples, for example tissue samples.

There is a need for identification of proteins in complex mixtures as well as for the detection of differences in relative expression profiles. A given protein is considered present in a sample when sufficient numbers of its peptides have been identified. It is known in the art to use MSMS (tandem mass spectrometry) for fast and parallel identification of a large number of peptides. First, a fragmentation pattern, i.e. a spectrum of a peptide is generated using a mass spectrometer, and on the basis of the generated spectrum the peptide sequence is identified. This process is basically performed as follows, in brief. Subsequent to separation which reduces sample complexity (for example with liquid chromatography), digestion with an appropriate enzyme (e.g., trypsin) generates the peptides to be detected. Then using a mass spectrometer, a mass-based selection is performed, and in a second chamber of mass spectrometer a collision-induced dissociation is performed so that fragmentation takes place. Due to the collision with inert gas in the second spectrometer chamber, the peptides break into pieces and a plurality of fragments is obtained having a mass from 0 up to the mass of an unbroken peptide. For identification, the fragmentation spectrum is then connected to a sequence. Thus, a sequence (or a part of it) can be read from the spectrum. Finally, a database search is necessary, performing spectral comparisons using the experimental spectrum until the best match is found. That is, the fragmentation spectrum of peptide is compared against theoretically generated spectra of candidate peptides. Due to the high number of data produced by this comparison, the post processing of the data is very time-intensive. This limits the extent of the experiment beforehand. The post-processing of data resulting from comparing or manipulating results from different experiments becomes very difficult and time-consuming as no practical solution exists to deal with the huge number of generated data.

It is therefore an object of the invention to provide a method and system to improve and accelerate the post processing of the peptides, i.e. the allocation of the identified peptides to proteins and protein groups. This object is achieved with the features of the claims.

A first aspect of the invention relates to a method of processing protein peptide data obtained from healthy or pathological samples for analysis, comprising the steps of: (a) providing a list of peptide sequences and associated auxiliary information representing an input data set; (b) compiling from the input data set a new peptide sequence list by removing peptide sequence redundancy in the peptide sequence list, said new peptide sequence list representing a peptide data set; (c) and grouping together members of the peptide data set originating from the same protein thus generating a protein data set.

The auxiliary information preferably comprises at least one of the following: corresponding metric values, originating protein, physicochemical properties of the peptide, the offset of the peptide in the protein sequence.

In step b) a peptide redundancy is preferably represented in the new peptide sequence list by a single entry. The peptide metric value of the single entry is preferably calculated by taking into account the corresponding values of all redundant peptide sequences.

Step c) preferably comprises calculating overall protein metrics for each protein based on the measured values of each of its peptides.

The input data sets, protein data sets, and peptide data sets are preferably stored in a relational database. Each peptide sequence is for example mapped to a unique number, and the sum of the unique numbers of the peptides of one protein provides a unique identification number for each protein. It is preferred that the grouping is based on these unique identification numbers.

According to a preferred embodiment, at least some of the data sets are visualized.

The method of the first aspect preferably further comprises the steps of (d) determining and grouping within a protein data set proteins sharing identical peptides thus forming protein group data sets; and thereby detecting redundancy within the protein set.

According to the invention, two data sets are provided and processed, one for the healthy tissue and one for diseased tissue, in order to find those portions in the diseased tissue that cannot be found in the healthy tissue. Thus, as a result the invention provides a list of proteins that are present in the diseased tissue but not in the healthy tissue or vice-versa.

A second aspect of the invention relates to a method comprising the steps of (a) providing at least two peptide data sets or protein data sets relating to healthy or diseased tissue; (b) merging said peptide data sets or protein data sets to generate a composite data set; and (c) outputting the composite data set.

According to the method of the second aspect, the peptide data sets or protein data sets of healthy tissue are preferably merged with other peptide data sets or protein data sets of healthy tissue. Alternatively, peptide data sets or protein data sets of diseased tissue are merged with other peptide data sets or protein data sets of diseased tissue. As a further alternative, peptide data sets or protein data sets of healthy tissue are merged with peptide data sets or protein data sets of diseased tissue.

The merging in step (b) is preferably performed according to rules of Boolean operations and combinations thereof. Preferably, in the merging step the various metrics for each member protein or member peptide are calculated in order to include the contributions from each original data set.

According to a preferred option, the method of the second aspect further comprises the step of merging a first composite data set with at least one further composite data set to generate a higher generation composite data set.

The peptide data sets are preferably obtained by providing a list of peptide sequences and associated auxiliary information representing an input data set; and compiling from the input data set a new peptide sequence list by removing peptide sequence redundancy in the peptide sequence list, said new peptide sequence list representing a peptide data set.

The protein data sets are preferably obtained by providing a list of peptide sequences and associated auxiliary information representing an input data set; compiling from the input data set a new peptide sequence list by removing peptide sequence redundancy in the peptide sequence list, said new peptide sequence list representing a peptide data set; and grouping together members of the peptide data set originating from the same protein thus generating a protein data set.

It is preferred according to the first or second aspect to generate a restricted peptide data set or protein data set from a single peptide data set or protein data set by excluding those members that do not meet preset criteria. The preset criteria may be user input criteria. The criteria for peptide set restriction are, for example, metric thresholds, sequence features such as presence or absence of specific amino acids, mass constraints, or constraints on other physicochemical properties. Furthermore, criteria for protein set restriction are, for example, metric thresholds, sequence content of the protein, physicochemical properties.

The method of the first or second aspect preferably comprises the step of comparing a first protein data set and a second protein data set to determine the degree ot similarity between the protein expression patterns of the two protein sets. The comparison may be performed by using a statistical rank correlation test, for example on the number of peptide counts of the common proteins, or alternatively on the different detected peptides per protein. The statistical rank correlation test may also be performed on the protein coverage.

The result of the comparison contains information about protein abundance patterns.

The invention also provides a system for processing protein peptide data obtained from healthy or pathological samples for analysis, comprising means for providing a list of peptide sequences and associated auxiliary information representing an input data set; means for compiling from the input data set a new peptide sequence list by removing peptide sequence redundancy in the peptide sequence list, said new peptide sequence list representing a peptide data set; and means for grouping together members of the peptide data set originating from the same protein thus generating a protein data set.

Furthermore, the invention provides a system comprising means for providing at least two peptide data sets or protein data sets relating to healthy or diseased tissue; means for merging said peptide data sets or protein data sets to generate a composite data set; and means for outputting the composite data set.

The invention will now be explained with reference to the accompanying figures, in which:
- Fig.: 1 is a schematic overview showing the method according to a preferred embodiment of the first aspect of the invention and preferred additional steps;
- Fig. 2 is: a visualization of data structure dependencies;
- Fig. 3: shows an example of a non-redundant peptide list constituting a peptide set; and
- Fig.: 4 shows an example of a protein set.

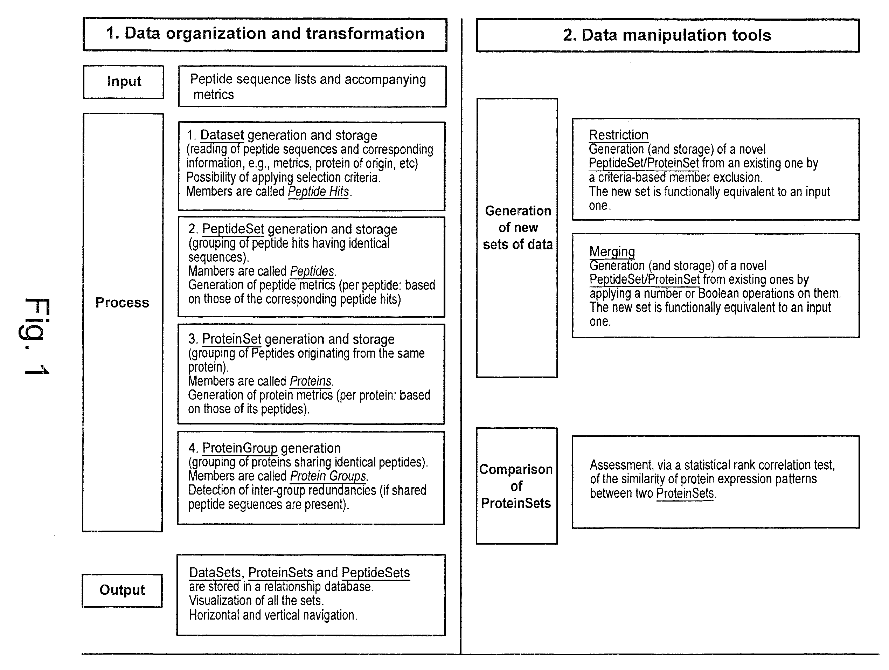

According to the method of the first aspect of the invention, input data are provided such as a list of peptide sequences and associated auxiliary information. The list of peptide sequences and the associated auxiliary information represent an input data set (see Fig. 1 "Input").

For each experiment, the invention stores in appropriately designed data structures the input sequences and relevant information such as the corresponding metrics values, the originating protein, etc. An input sequence is a single peptide sequence (the terms peptide and sequence are used interchangeably; a peptide is uniquely identified by its sequence). Each such peptide belongs to a protein but it is not at all necessary that all possible peptides of a protein present in the sample are part of the input (i.e., detected in an experiment). These peptide sequences (the ones most likely to be present in the experimental sample) are determined in a preceding process based on various criteria. The additional information may include: various metrics derived by the preceding annotating algorithm(s) that quantify the likelihood that the annotation (the actual decision process that the given peptide was indeed in the sample) is incorrect, various physicochemical properties of the peptide, its offset in the protein sequence, the name of the actual computer data file where the annotating routine stored the results, information concerning the overall experimental design/procedures, the name of the user, etc.

The members of such input data set are called peptide hits.

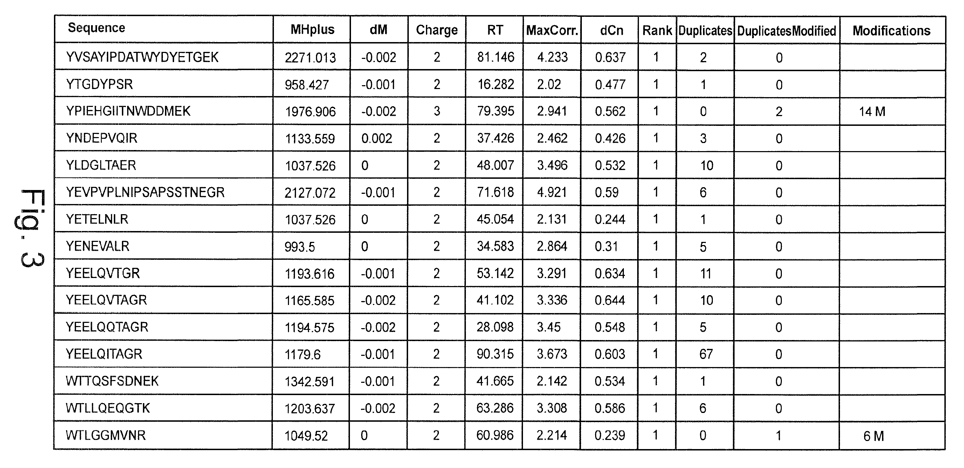

On the basis of the input data set, a new peptide sequence list is compiled or generated by removing peptide sequence redundancy (a set of identical peptide sequences) in the peptide sequence list. The new peptide sequence list represents a peptide data set, and the members of such peptide data set are called peptides. A peptide redundancy is then represented by a single entry, whose accompanying measured values are calculated by taking into account the corresponding values of all the redundant members. This new, non-redundant peptide list constitutes the peptide set. This is shown by means of an example in Fig. 3. Each row corresponds to a peptide which may have been identified multiple times. This is shown at the columns "Duplicates" and "DuplicatesModified". For example the peptide in the first row has been identified 2 times ("2" in column "Duplicate" + "0" in column "DuplicatesModified"), the second once, the third twice. In more detail, this means that the first sequence has been identified 2 times, there are 2 entries in the input data set of it. At the current level of the peptide set these 2 entries have been consolidated to just one, i.e., all peptide redundancy has been removed and the two aforementioned columns provide information about that redundancy.

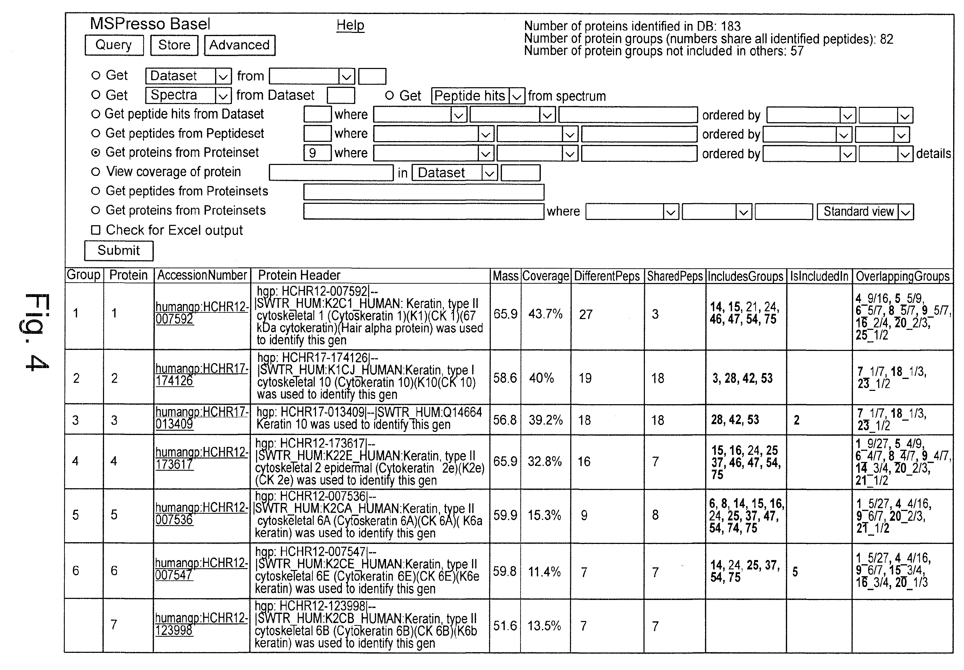

The next step or level of the invention is the generation of the so-called protein set. The protein set is a list of proteins generated by grouping together peptides (members of the peptide set) originating from the same protein (as defined in the sequence database). This is shown in Fig. 4. This step also entails the calculation of various metrics for each protein, based on the measured values of the corresponding peptides. Such a metric is the so called protein coverage. This is the percentage of the sequence of the protein that has been annotated in the experiment. In more detail, an experiment detects peptides, and these correspond to protein fragments. A given experiment may result in thousands of detected peptides that are mapped to a set of proteins (could be thousands of them too). A protein could, in principle, be represented in the experiment by all its possible fragment peptides (100% coverage). However, only a subset of them is detected reliably thus resulting in a smaller coverage. For each protein set member all its detected peptides are grouped together and subsequently used to calculate the percentage coverage (ratio of the length of protein sequence "seen" in the experiment over the total length of the protein sequence). Such calculation has to take into account all the possibilities of the eventual overlaps of the various detected protein subsequences (i.e., peptide sequences) in order to avoid double counting, etc. Another metric that may be calculated in this context is the so-called "protein score". This is usually, in a nutshell, a measure of the likelihood that for a given protein the annotating algorithm(s) include in their output peptides of it despite the fact that the protein is not present in the experimental sample. The reason is that each algorithm produces wrong identifications no matter how accurate it claims to be. These wrong identifications propagate, of course, to the protein level. All algorithms, however, provide metrics to help quantify the likelihood that such a wrong identification may occur. Each identified peptide is accompanied by such metrics. For each protein, it is preferred according to the invention to combine the metrics of all its identified peptides and to generate an overall metric for the protein. Such protein set is shown in Fig. 4. The table shown in Fig, 4 provides as additional output information the coverage of the protein, the number of different peptides, and the number of shared peptides. Furthermore, information is provided about group overlaps. For example, group "3" also includes groups "28", "42", and "53", and is itself included in group "2".

According to a preferred embodiment, the method comprises the optional steps of determining and grouping within a protein data set proteins sharing identical peptides thus forming protein group data sets; and thereby detecting redundancy within the protein set. In this preferred aspect of the invention, any members of a protein set whose sets of detected peptides are identical are named members of the same protein group. This can also be seen in Fig. 4 with respect to group 6 which contains data entries for protein 6 and protein 7. The presence of non-trivial protein groups (groups with more than one member) signals a redundancy in the sequence database used in the experiment. This simply mirrors the fact that the experimental procedure employed is unable to distinguish whether one or more members of a given protein group are actually present in the sample under analysis. This optional highest part of the data organization is thus referred to as a protein group.

Alternatively, it would be possible to perform such grouping already at the level of peptides.

Finally, the input data sets, protein data sets, and peptide data sets are stored in a relational database for output to and access by the user (see Fig. 1 "Output").

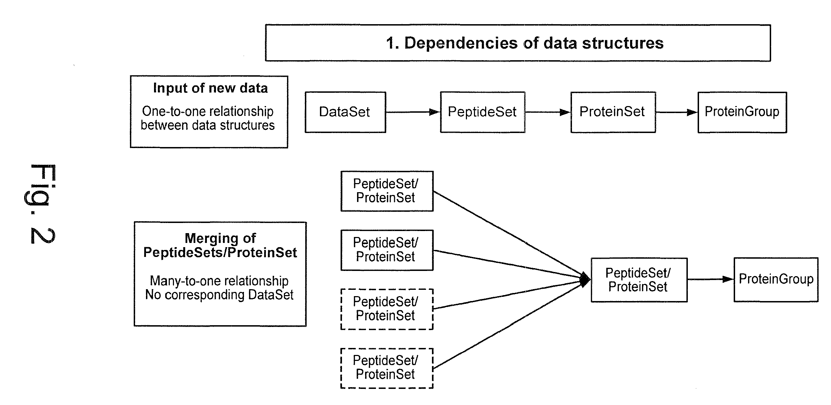

The data structure of the method described so far is shown in the upper diagram of Fig. 2. There is a one-to-one relationship between the Data set, the peptide set, the protein set, and the optional protein group.

On the basis of the results obtained with the method according to the first aspect of the invention, new data sets are preferably generated, for example by restriction. By restriction is meant the generation of a new peptide set or protein set from a single peptide set or protein set through an exclusion of those members of the older set that do not meet preset criteria, for example user-input criteria. Possible criteria for a peptide set restriction are threshold values. Each peptide set member is characterized by metrics quantifying the quality of the original annotation process. By imposing threshold values on such metrics functional subsets of the original peptide set can be produced, e.g., new peptide sets. Another way is to keep only peptides with specific sequence features (e.g., presence or absence of specific amino acids), enforce mass constraints (keep only peptides with mass larger/smaller than a given value, etc) as well as constraints on other physicochemical properties. With respect to protein set restriction, the invention encompasses the generation from a given protein set new functional protein set(s) by enforcing metrics thresholds (e.g., on protein coverage and/or on protein score), constraints on the sequence content of the protein, on physicochemical properties (mass, isoelectric point, etc) as well as on relevant biological information (e.g., keep only proteins active in a certain pathway or expressed only in a certain organelle, tissue, etc).

According to a further aspect of the invention, new sets of data are generated by a merging step. By merging is meant the generation of a new peptide set or protein set from a multitude of peptide sets or protein sets. The rules of merging can be any possible combination of Boolean operations on the different sets. In all, merging operations the various metrics for each member peptide/protein are calculated in order to take include the contributions from each original set.

Thus a method according to the second aspect of the invention comprises the steps of providing at least two peptide data sets or protein data sets relating to healthy or diseased tissue, merging said peptide data sets or protein data sets to generate a composite data set; and outputting the composite data set. For example, peptide data sets or protein data sets of healthy tissue are merged with other peptide data sets or protein data sets of healthy tissue. Alternatively, peptide data sets or protein data sets of diseased tissue are merged with other peptide data sets or protein data sets of diseased tissue. As a further alternative, peptide data sets or protein data sets of healthy tissue are merged with peptide data sets or protein data sets of diseased tissue.

The data structure dependency with respect to merging of peptide sets or protein sets is shown in the lower diagram in Fig. 2. For example four peptide sets or protein sets are merged to a single set which may then also undergo a grouping step, as described above. This scenario represents a many-to-one relationship with no corresponding input data set.

Any peptide set or protein set that has been generated by either restriction or merging is designated as *composite.* A composite set does not correspond directly to a data set. However, the way the data is structured and stored allows to connect any composite set to the corresponding data set(s) of its generating peptide set(s) or protein set(s).

According to a further option of the present invention restriction and/or merging can be further applied on such composite peptide sets/protein sets thus generating second (or higher) generations of new composite sets. It is always possible to connect them to the original generating peptide set(s)/protein set(s) (and, of course, data set(s)). Furthermore, for each composite proteins set the system of the invention may preferably generate the corresponding protein group.

These two ways of generating new sets of data are shown in the right part of Fig. 1. It is preferably to first perform the merging step in order to keep all information, and to then perform a restriction to further limit the amount of data.

According to a further preferred embodiment, the invention provides a comparison of experiments (see right part of Fig. 1). By comparison of two experiments is meant the estimation of the similarity between their observed protein abundance patterns. For a single protein, the measure of its abundance is taken to be the number of its experimentally identified peptide sequences (peptide counts). The comparison of the protein abundance patterns is sensible only when the number of the common proteins of the two protein sets is sufficiently large. When this is true, then a statistical rank correlation test, for example, is perfomed on the peptide counts of the common proteins, providing a robust measure of the similarity between the observed abundance patterns. Protein abundance patterns correspond, to a large extent, to the number of identified peptides per protein. In two similar samples a protein is expected to be present in comparable concentrations, which in turns means that the number of its corresponding detected peptides in two parallel experiments should be comparable. The (e.g., Spearman) statistical rank correlation test creates a paired list of the detected peptides per protein in the two experiments and then generates a statistically meaningful value that indicates whether the protein abundance patterns in one protein set are mirrored in the other.

Thus, the invention provides the ability to restrict or merge sets of data generating new functional ones as well as enabling comparisons based on various measurable properties. As an example, comparison is performed only between protein sets and the statistical rank correlation test is based exclusively on the number of detected peptides per protein. However, the invention encompasses other parameters on which such comparison could be based, e.g. the number of different detected peptides per protein (this number is equal or smaller to the number of the detected peptides per protein, the former counts as one all present multiple detections (duplicates) of the same peptide, while the latter counts them as independent ones). Yet another such parameter to use for comparison is the protein coverage.

Although specific embodiments of the present invention have been described above in detail, it will be understood that this description is merely for purposes of illustration. Various modifications of and equivalents corresponding to the disclosed aspects of the preferred embodiments described above may be made by those skilled in the art without departing from the spirit of the present invention, which is defined by the following claims.

## Claims

1. Method of processing protein peptide data obtained from healthy or pathological samples for analysis, comprising the steps of:
a) providing a list of peptide sequences and associated auxiliary information representing an input data set;
b) compiling from the input data set a new peptide sequence list by removing peptide sequence redundancy in the peptide sequence list, said new peptide sequence list representing a peptide data set; and
c) grouping together members of the peptide data set originating from the same protein thus generating a protein data set.

2. The method of claim 1, wherein the auxiliary information comprises at least one of the following: corresponding metric values, originating protein, physicochemical properties of the peptide, the offset of the peptide in the protein sequence.

3. The method of claim 1 or 2, wherein in step b) a peptide redundancy is represented in the new peptide sequence list by a single entry.

4. The method of claim 3, wherein the peptide metric value of the single entry is calculated by taking into account the corresponding values of all redundant peptide sequences.

5. The method of any of the preceding claims, wherein step c) comprises calculating overall protein metrics for each protein based on the measured values of each of its peptides.

6. The method of any of the preceding claims, further comprising storing the input data sets, protein data sets, and peptide data sets in a relational database.

7. The method of claim 6, wherein each peptide sequence is mapped to a unique number, and the sum of the unique numbers of the peptides of one protein provides a unique identification number for each protein.

8. The method of claim 7, wherein grouping is based on the unique identification numbers.

9. The method of any of the preceding claims, further comprising visualizing of at least some of the data sets.

10. The method of any of the preceding claims, further comprising:
determining and grouping within a protein data set proteins sharing identical peptides thus forming protein group data sets, and thereby detecting redundancy within the protein set.

11. A method comprising the steps of:
a) providing at least two peptide data sets or protein data sets relating to healthy or diseased tissue;
b) merging said peptide data sets or protein data sets to generate a composite data set; and
c) outputting the composite data set.

12. The method of claim 11, wherein peptide data sets or protein data sets of healthy tissue are merged with other peptide data sets or protein data sets of healthy tissue.

13. The method of claim 11, wherein peptide data sets or protein data sets of diseased tissue are merged with other peptide data sets or protein data sets of diseased tissue.

14. The method of claim 11, wherein peptide data sets or protein data sets of healthy tissue are merged with peptide data sets or protein data sets of diseased tissue.

15. The method of any of claims 11 to 14, wherein the merging in step b) is performed according to rules of Boolean operations and combinations thereof.

16. The method of any of claims 11 to 15, wherein in the merging step the various metrics for each member protein or member peptide are calculated in order to include the contributions from each original data set.

17. The method of any of claims 11 to 16, further comprising the step of merging a first composite data set with at least one further composite data set to generate a higher generation composite data set.

18. The method of any of claims 11 to 17, wherein the peptide data sets are obtained by providing a list of peptide sequences and associated auxiliary information representing an input data set; and compiling from the input data set a new peptide sequence list by removing peptide sequence redundancy in the peptide sequence list, said new peptide sequence list representing a peptide data set.

19. The method of any of claims 11 to 17, wherein the protein data sets are obtained by providing a list of peptide sequences and associated auxiliary information representing an input data set; compiling from the input data set a new peptide sequence list by removing peptide sequence redundancy in the peptide sequence list, said new peptide sequence list representing a peptide data set; and grouping together members of the peptide data set originating from the same protein thus generating a protein data set.

20. The method of any of claims 1 to 19, further comprising generating a restricted peptide data set or protein data set from a single peptide data set or protein data set by excluding those members that do not meet preset criteria.

21. The method of claim 20, wherein the preset criteria are user input criteria.

22. The method of claim 20 or 21, wherein criteria for peptide set restriction are metric thresholds, sequence features such as presence or absence of specific amino acids, mass constraints, or constraints on other physicochemical properties.

23. The method of claim 20 or 21, wherein criteria for protein set restriction metric thresholds, sequence content of the protein, physicochemical properties.

24. The method of any of claims 1 to 23, further comprising the step of comparing a first protein data set and a second protein data set to determine the degree of similarity between the protein expression patterns of the two protein sets.

25. The method of claim 24, wherein the comparison is performed by using a statistical rank correlation test.

26. The method of claim 25, wherein the statistical rank correlation test is performed on the number of peptide counts of the common proteins.

27. The method of claim 25, wherein the statistical rank correlation test is performed on the different detected peptides per protein.

28. The method of claim 25, wherein the statistical rank correlation test is performed on the protein coverage.

29. The method of claim 25 or 26, wherein the result of the comparison contains information about protein abundance patterns.

30. System for processing protein peptide data obtained from healthy or pathological samples for analysis, comprising:
a) means for providing a list of peptide sequences and associated auxiliary information representing an input data set;
b) means for compiling from the input data set a new peptide sequence list by removing peptide sequence redundancy in the peptide sequence list, said new peptide sequence list representing a peptide data set; and
c) means for grouping together members of the peptide data set originating from the same protein thus generating a protein data set.

31. System comprising:
a) means for providing at least two peptide data sets or protein data sets relating to healthy or diseased tissue;
b) means for merging said peptide data sets or protein data sets to generate a composite data set; and
c) means for outputting the composite data set.
